# EUROPÄISCHE PATENTSCHRIFT

(11) **EP 1 299 541 B1**
(45) Veröffentlichungstag und Bekanntmachung des Hinweises auf die Patenterteilung: **12.09.2007**
(21) Anmeldenummer: 01969365.4
(22) Anmeldetag: 11.07.2001
(51) Int. Cl.: C12N 15/12

(54) **VERFAHREN ZUR GEWINNUNG UND ANWENDUNG NEUER HUMANER DEFENSINE ALS BIOLOGISCH AKTIVE EIWEISSTOFFE ZUR BEHANDLUNG VON INFEKTIONEN UND ANDEREN ERKRANKUNGEN**
METHOD FOR PRODUCING AND USING NOVEL HUMAN DEFENSINS AS BIOLOGICALLY ACTIVE PROTEINS FOR TREATING INFECTIONS AND OTHER ILLNESSES
PROCEDE D'EXTRACTION ET D'UTILISATION DE NOUVELLES DEFENSINES HUMAINES EN TANT QUE PROTEINES BIOLOGIQUEMENT ACTIVES POUR TRAITER DES INFECTIONS ET AUTRES PATHOLOGIES

(30) Priorität: 11.07.2000 DE 10033505
(43) Veröffentlichungstag der Anmeldung: 09.04.2003
(73) Patentinhaber: IPF Pharmaceuticals GmbH, 30625 Hannover (DE)
(72) Erfinder: FORSSMANN, Wolf-Georg, 30175 Hannover (DE); CONEJO-GARCIA, Jose-Ramon, 30625 Hannover (DE); ADERMANN, Knut, 30625 Hannover (DE)
(74) Vertreter: Meyers, Hans-Wilhelm
(86) Internationale Anmeldenummer: PCT/EP2001/007973
(87) Internationale Veröffentlichungsnummer: WO 2002/004487

(56) Entgegenhaltungen:
- WO-A-00/46245
- WO-A-99/13080
- JR CONEJO GARCIA ET AL.: "Human beta defensin 4: a novel inducible peptide with a specific salt-sensitive spectrum of antimicrobial activity" FASEB JOURNAL, Bd. 15, Nr. 10, 2001, Seiten 1819-1821, XP002201162
- DIAMOND G. AND BEVINS C.L.: "beta Defensins: Endogenous Antibiotics of the Innate Host Defense Response" CLINICAL IMMUNOLOGY AND IMMUNOPATHOLOGY, Bd. 88, Nr. 3, September 1998 (1998-09), Seiten 221-225, XP002201163

### SEQUENZPROTOKOLL

<110> IPF Pharmaceuticals GmbH
<120> Verfahren zur Gewinnung und Anwendung neuer humaner Defensine als biologisch aktive Eiweißstoffe zur Behandlung von Infektionen und anderen Erkrankungen
<130> 011666wo
<140> PCT/EP01/07973
   <141> 2001-07-11
<150> DE10033505.5
   <151> 2000-07-11
<160> 78
<170> PatentIn Ver. 2.1
<210> 2
   <211> 29
   <212> PRT
   <213> Homo sapiens
<400> 2
   Cys Gly Tyr Gly Thr Ala Arg Cys Arg Lys Lys Cys Arg Ser Gln Glu
     1               5                  10                  15
   Tyr Arg Ile Gly Arg Cys Pro Asn Thr Tyr Ala Cys Cys
                20                  25
<210> 54
   <211> 151
   <212> DNA
   <213> Homo sapiens
<400> 54
   cgaatttgaa ttggacagaa tatgtggtta tgggactgcc cgttgccgga agaaatgtcg 60
   cagccaagaa tacagaattg gaagatgtcc caacacctat gcatgctgtt tgagaaaatg 120
   ggatgagagc ttactgaatc gtacaaaacc c                                151

## Beschreibung

Die Erfindung betrifft das Peptid hBD-6 vom human Defensintyp, ein Verfahren zur Gewinnung erfindungsgemäßer Peptide in reiner oder partiell aufgereinigter Form aus menschlichen und tierischen Körperflüssigkeiten, die die Fähigkeit besitzten, die bakterielle Invasion bei Entzündungserkrankungen zu verhindern, Nukleinsäuren, die für diese Peptide kodieren, Arzneimittel enthaltend diese Peptide, sowie Verwendungen dieser Peptide zur Behandlung verschiedener Erkrankungen.

Diese Peptide lassen sich insbesondere aus Haemofiltrat oder Haemodialysat aus menschlichem und tierischem Blut gewinnen. Diese Stoffe sind als humane Defensine klassifiziert und können zum Zwecke (1) der medizinischen und gewerblichen Verwendung als Medikament und (2) der Analyse von Erkrankungen benutzt werden.

Der Stoff mit der Kurzbezeichnung hBD-6 (humanes beta-Defensin-6) wurde erstmals aus dem Haemofiltrat Nierenkranker nach Ultrafiltration am Haemodialyseapparat gewonnen und über einen antibakteriellen Hemmtest funktionell charakterisiert. Zur Darstellung der Defensinpeptide wurde ein patentiertes Verfahren (Forssmann, 1988; DE 3633707 C1) verfeinert, welches zuvor für Gewinnung von Eiweißstoffen aus Haemofiltrat erfunden wurde. Aus den mit diesem Verfahren gewonnenen Molekülen mit einem Molekulargewicht unter 20 kDalton, die bei veno-venöser oder arterio-venöser Shuntverbindung abfiltriert werden, können die Peptidfraktionen enthaltend die humanen Defensinpeptide durch einen Funktionstest erkannt werden. Das bisher bekannte Verfahren wurde benutzt, um die Rohpeptidextrakte zu gewinnen, mit denen bei der Anwendung des LEHRERschen Radialdiffusionstest ein starker Effekt festgestellt wurde, indem das Wachstum von Bakterien in Kultur unter dem Einfluss dieser Substanz stark gehemmt wird.

Es wurde weiter festgestellt, dass bei weiteren Reinigungsverfahren diese biologischen Aktivitäten konzentriert werden konnten, bis schließlich verschiedene einheitliche Eiweißstoffe identifiziert und in ihrer Struktur aufgeklärt wurden. Vorteilhafterweise können diese Stoffe aus dem bisher als wertlos betrachteten Haemofiltrat aufgereinigt werden, um als wirtschaftlich verwertbare Substanzen benutzt zu werden. Die erfindungsgemäßen Peptide lassen sich durch chemische Synthese und durch gentechnologische Produktion gewinnen, sie lassen sich einsetzen u.a. als pathognomonisches Diagnosemerkmal für die Analyse von entzündlichen Erkrankungen des Magen-Darm-, Respirations- und Urogenitaltraktes sowie anderer Epithelorgane.

Die vorliegende Erfindung betrifft ein Peptid mit der Aminosäuresequenz
(b) hBD-6
   Z_{N3}-CGYGTARCRKKCRSQEYRIGRCPNTYACC-Z_{C3}

Die folgenden vier Peptide sind nicht Gegenstand der Erfindung und wurden lediglich zu Vergleichszwecken aufgeführt:
(e) hBD-10
   -CHMQQGICRLFFCHSGEKKRGICSDPWNRCC-
(k) hBD-16
   -CWNNYVQGHCRKICRVNEVPEALCENGRYCC-
(l) hBD-17
   -CWNLYGKCRYRCSKKERVYVYCINNKMCC-
(n)hBD-19
   -CLMGLGRCRDHCNVDEKEIQKCKMKKCC-
wobei,
Z_{N3} einen Aminosäurerest oder einen Peptidrest von bis zu 30 Aminosäuren, insbesondere den Peptidrest EFELDRI und seine N-terminal verkürzten Fragmente, bedeutet,
Z_{C3} einen Aminosäurerest oder einen Peptidrest von bis zu 30 Aminosäuren, insbesondere den Peptidrest LRKWDESLLNRTKP und seine C-terminal verkürzten Fragmente, bedeutet,
und die zyklischen, amidierten, acetylierten, sulfatierten, phosphorylierten, glycosylierten, und oxydierten Derivate mit antibiotischer Aktivität, die aus den oben beschriebenen Aminosäuresequenzen abgeleitet werden.

Für die oben beschriebenen neuen Defensinpeptide wurde folgende kodierende Nukleinsäuresequenz (cDNA) gefunden, die auch Gegenstand der vorliegenden Erfindung ist:
(b) hBD-6

Durch die Analyse der kodierenden Nukleotidsequenz wurde das Gen des neuen Defensinpeptids hBD-6 auf Chromosom 8 gefunden.

Damit ist weiter Aufgabe der vorliegenden Erfindung, das neue Peptid hBD-6 bereitzustellen, das dadurch gekennzeichnet ist, dass es jeweils als ein gut zugängliches Arzneimittel mit biologisch und therapeutischer Aktivität eines natürlichen Stoffes verwendet werden kann.

Die vorliegende Erfindung stellt des weiteren ein Herstellungsverfahren für die erfindungsgemäßen Peptide sowie die Verwendung der erfindungsgemäßen Peptide als Arzneimittel für verschiedene therapeutische und diagnostische Indikationen bereit. Dazu können die Defensinpeptide als hochreine Stoffe oder - wenn für die bestimmte Verwendung ausreichend - innerhalb eines teilweise aufgereinigten Peptidgemisches oder als Gemisch mehrerer der erfindungsgemäßen hochreinen Defensinpeptide verwandt werden.

Die erfindungsgemäßen Peptide können eingesetzt werden zur Behandlung von Erkrankungen, die bei bakteriellen Organbesiedlungen entstehen.

Die erfindungsgemäßen Peptide sind weiterhin einsetzbar zur Behandlung von Erkrankungen des menschlichen Organismus, insbesondere mit Beteiligung des Magen-Darm-Traktes, der Atemwege und des Urogenitalapparates.

Die erfindungsgemäßen Peptide können in einer weiteren Ausgestaltung der Erfindung eingesetzt werden zur Behandlung von Erkrankungen des menschlichen Organismus, insbesondere mit Beteiligung des Intugementes und seiner Anhangsdrüsen.

Die erfindungsgemäßen Peptide können auch eingesetzt werden zur Behandlung von Systemerkrankungen bei Überproduktion oder Mangel der Defensinpeptide, insbesondere durch gegen die Defensinpeptide gebildete Antikörper oder zur Verwendung in der Substitutionstherapie.

In einer weiteren Ausführungsform der Erfindung können die erfindungsgemäßen Peptide zur Behandlung von chronischen Erkrankungen, teils vergesellschaftet mit den bereits erwähnten Erkrankungen eingesetzt werden, indem diese in geeigneter Form für die Behandlung benutzt werden.

Die erfindungsgemäßen Peptide können weiterhin eingesetzt werden zur Behandlung von Erkrankungen im akuten Stadium.

Die erfindungsgemäßen Peptide können eingesetzt werden zur Behandlung der Störung der Fertilität, insbesondere bei Krankheiten der mit Oocyten verbundenen Spermienpenetrationsstörungen und Inidationsstörungen sowie Maturationsstörungen im männlichen Reproduktionsapparates, sowie als Kontrazeptivum.

Die erfindungsgemäßen Peptide können eingesetzt werden zur Diagnose der bereits erwähnten Erkrankungen, indem beispielsweise Antikörper gegen eines oder mehrere der erfindungsgemäßen Peptide oder seiner Derivate oder ihrer Fragmente hergestellt werden und die Blutkonzentration eines oder mehrerer der erfindungsgemäßen Peptide über immunologische Verfahren gemessen wird.

Die vorliegende Erfindung betrifft weiter verschiedene Verfahren zur Herstellung der erfindungsgemäßen neuen Defensinpeptide oder ihrer Derivate dadurch gekennzeichnet, dass dieses über eine prokaryontische oder eine eukaryontische Expression hergestellt und chromatographisch gereinigt werden, sowie ein weiteres Verfahren zur Herstellung der Defensinpeptide ihrer Derivate, indem man sie aus menschlichem Blut über Chromatographie-Verfahren in bekannter Weise isoliert, und schließlich ein Verfahren zur Herstellung der Defensinpeptide oder ihrer Derivate, indem man diese Defensinpeptide durch die üblichen Verfahren der Festphasen- und Flüssigphasen-Synthese aus den geschützten Aminosäuren, die in der angegebenen Sequenz enthalten sind, herstellt, deblockiert und es mit den gängigen Chromatographie-Verfahren reinigt.

Die Defensinpeptide werden chemisch synthetisiert und als Arzneimittel zubereitet. Auch die gentechnologische Herstellung durch Verwendung übliche Vektoren ist erarbeitet. Auf diesem Wege wird die neuen Defensinpeptide sowohl (1) in prokaryontischen als auch (2) in eukaryontischen Organismen hergestellt. Hierfür stehen verschiedene Expressionsvektoren routinemässig zur sekretorischen oder direkten cytoplasmatischen Expression zur Verfügung.

Die Arzneimittelzubereitungen enthalten eines oder mehrere der erfindungsgemäßen neuen Defensinpeptide oder ein physiologisch verträgliches Salz dieser Peptide. Die Form und Zusammensetzung der Arzneimittel, welche eines oder mehrere der neuen Defensinpeptide enthalten, richtet sich nach der Art der Verabreichung. Die Arzneimittel eines oder mehrere der neuen Defensinpeptide enthaltend können parenteral, intranasal, oral und mittels Inhalation verabreicht werden. Vorzugsweise werden diese Arzneimittel enthaltend eines oder mehrere der neuene Defensinpeptide mit einem Injektionspräparat, entweder als Lösung oder als Lyophilisat zur Auflösung unmittelbar vor Gebrauch konfektioniert. Die Arzneimittelzubereitungen können außerdem Hilfsstoffe enthalten, die abfülltechnisch bedingt sind, einen Beitrag zur Löslichkeit, Stabilität oder Sterilität des Arzneimittels leisten oder den Wirkungsgrad der Aufnahme in den Körper erhöhen.

Die zu verabreichende Tagesdosis für die erfindungsgemäßen Defensinpeptide hängt von der Indikation und der Anwendung bestimmter Derivate ab. Bei i.v./i.m. Injektion liegt sie im Bereich von 100 bis 1200 Einheiten (µg)/Tag, bei täglicher subcutaner Injektion vorzugsweise bei 300 - 2400 Einheiten (µg)/Tag.

Die Bestimmung der biologischen Aktivität für die erfindungsgemäßen neuen Defensinpeptide basiert auf Messungen gegen international gebräuchliche Referenzpräparationen für antibiotische Substanzen.
Das erfindungsgemäße neue Defensinpeptid hBD-6 ist dadurch gekennzeichnet, dass es sich besonders auch für die Langzeit-Therapie bei Infektionserkrankungen eignet, da es über eine ausgezeichnete biologische Wirksamkeit verfügt und andererseits auch bei Dauerbehandlung keine Immunreaktion auslöst.

Aufgrund der biologischen Wirkung der erfindungsgemäßen Defensinpeptide ist gezeigt, dass die erfindungsgemäßen Präparate weiter als Mittel zur Therapie von infektiösen Erkrankungen vieler Epithelorgane anwendbar sind.

Zur Bestimmung der Aktivität wurden beispielhaft die nicht erfindungsgemäßen Peptide hBD10, hBD17 und hBD19 auf ihre antimikrobielle Wirkung hin getestet. Im Radial-Diffusions-Assay konnten die in Tabelle 1 angegebenen Aktivitäten der Peptide gegen verschiedene Bakterienstämme gemessen werden. Dabei bedeutet (+) die Bildung eines Hemmhofes und (-) keine Bildung eines Hemmhofs. Tabelle 1

| | hBD10 | hBD17 | hBD19 |
|---|---|---|---|
| Escherichia coli | (+) | (+) | (+) |
| Staphylococcus carnosus | (+) | (+) | (+) |
| Saccharomyces cerevisiae | (+) | (+) | (-) |

Für eine genauere Bestimmung der antibiotischen Aktivität wurde die minimale inhibitorische Konzentration (MIC) der o.g. Defensine nach Standardmethoden bestimmt. Die Befunde sind in Tabelle 2 angegeben, wobei die MIC-Werte Konzentrationen in [µg/ml] entsprechen (nd = nicht gemessen). Tabelle 2

| | hBD10 | hBD17 | hBD19 |
|---|---|---|---|
| Escherichia coli | nd | nd | nd |
| Staphylococcus carnosus | <50 | <25 | <25 |
| Saccharomyces cerevisiae | nd | nd | nd |

Weiterhin wurden Strukturanalysen mit dem nicht erfindungsgemäßen hBD16 durchgeführt. Abbildung 1 zeigt die in Lösung gefundene NMR-Struktur von hBD16.

Die räumliche Lage der Cysteine Cys 6, 15, 29 und 35 zeigt, dass die Verbrückung dieser Positionen nicht zwingend eine Strukturveränderung bedeuten muss, die zu einer Verminderung der Aktivität führt. Dieses konnte anhand des Vergleichs zweier Verbrückungsmuster gezeigt werden (Figur 2).

## Patentansprüche

1. Peptid mit der Aminosäuresequenz
(bb) hBD-6
Z_{N3}-CGYGTARCRKKCRSQEYRIGRCPNTYACC-Z_{C3},
wobei Z_{N3} einen Aminosäurerest oder einen Peptidrest von bis zu 30 Aminosäuren, insbesondere den Peptidrest EFELDRI und seine N-terminal verkürzten Fragmente und Z_{C3} einen Aminosäurerest oder einen Peptidrest von bis zu 30 Aminosäuren, insbesondere den Peptidrest LRKWDESLLNRTKP und seine C-terminal verkürzten Fragmente, bedeutet.

2. Peptid nach Anspruch 1, wobei das Peptid ein zyklisches, amidiertes, acetyliertes, sulfatiertes, phosphoryliertes, glycosyliertes oder oxydiertes Derivat, das aus den in Anspruch 1 beschriebenen Aminosäuresequenzen abgeleitet wird und antibiotische Aktivität aufweist, ist.

3. Verfahren zur Herstellung der Defensinpeptide oder ihrer Derivate nach mindestens einem der Ansprüche 1 bis 2, **dadurch gekennzeichnet, dass** diese über eine prokaryontische oder eine eukaryontische Expression hergestellt und gereinigt werden.

4. Verfahren zur Herstellung der Defensinpeptide oder ihrer Derivate nach mindestens einem der Ansprüche 1 bis 2, **dadurch gekennzeichnet, dass** man die Peptide aus menschlichem Blut über an sich bekannte übliche Chromatographie-Verfahren in bekannter Weise isoliert.

5. Verfahren zur Herstellung der Defensinpeptide oder ihrer Derivate nach mindestens einem der Ansprüche 1 bis 2, **dadurch gekennzeichnet, dass** man die Defensinpeptide oder ihre Derivate durch die üblichen Verfahren der chemischen Festphasen- und Flüssigphasen-Peptidsynthese aus den geschützten Aminosäuren, die in den angegebenen Sequenzen nach Anspruch 1 enthalten sind, herstellt, deblockiert und es mittels an sich bekannter Verfahren reinigt.

6. Arzneimittel, enthaltend eines oder mehrere der Defensinpeptide oder seiner Derivate nach mindestens einem der Ansprüche 1 bis 2 als aktiven Wirkstoff neben üblichen Hilfs- und Zusatzstoffen.

7. Verwendung eines oder mehrerer der Defensinpeptide oder seiner Derivate nach mindestens einem der Ansprüche 1 bis 2 zur Herstellung eines Arzneimittels zur Behandlung von Erkrankungen, die bei bakteriellen Organbesiedlungen entstehen.

8. Verwendung eines oder mehrerer der Defensinpeptide oder seiner Derivate nach mindestens einem der Ansprüche 1 bis 2 zur Herstellung eines Arzneimittels zur Behandlung von Erkrankungen des menschlichen Organismus, insbesondere mit Beteiligung des Magen-Darm-Traktes, der Atemwege und des Urogenitalapparates.

9. Verwendung eines oder mehrerer der Defensinpeptide oder seiner Derivate nach mindestens einem der Ansprüche 1 bis 2 zur Herstellung eines Arzneimittels zur Behandlung von Erkrankungen des menschlichen Organismus, insbesondere mit Beteiligung des Intugementes und seiner Anhangsdrüsen.

10. Verwendung eines oder mehrerer der Defensinpeptide oder seiner Derivate nach mindestens einem der Ansprüche 1 bis 2 zur Herstellung eines Arzneimittels zur Behandlung von Systemerkrankungen bei Überproduktion oder Mangel der Defensinpeptide, insbesondere durch gegen die Defensinpeptide gebildete Antikörper oder zur Verwendung der Defensinpeptide nach mindestens einem der Ansprüche 1 bis 2 zur Herstellung eines Arzneimittels zur Substitutionstherapie.

11. Verwendung eines oder mehrerer der Defensinpeptide oder seiner Derivate nach mindestens einem der Ansprüche 1 bis 2 zur Herstellung eines Arzneimittels zur Behandlung von chronischen Erkrankungen, teils vergesellschaftet mit Erkrankungen gemäß Ansprüchen 7 bis 10, indem es in geeigneter Form zur Herstellung eines Arzneimittels für die Behandlung benutzt wird.

12. Verwendung eines oder mehrerer der Defensinpeptide oder seiner Derivate nach mindestens einem der Ansprüche 1 bis 2 zur Herstellung eines Arzneimittels zur Behandlung von akuten Erkrankungen gemäß Ansprüchen 7 bis 11, indem es in geeigneter Form zur Herstellung eines Arzneimittels für die Behandlung in der Intensivpflege dieser Erkrankungen benutzt wird.

13. Verwendung eines oder mehrerer der Defensinpeptide oder seiner Derivate nach mindestens einem der Ansprüche 1 bis 2 zur Herstellung eines Arzneimittels zur Behandlung der Störung der Fertilität, insbesondere bei Krankheiten der mit Oocyten verbundenen Spermienpenetrationsstörungen und Nidationsstörungen sowie Maturationsstörungen im männlichen Reproduktionsapparates, sowie als Kontrazeptivum.

14. Verwendung eines oder mehrerer der Defensinpeptide oder seiner Derivate nach mindestens einem der Ansprüche 1 bis 2 zur Diagnose von Erkrankungen in vitro, insbesondere nach den Ansprüchen 7 bis 13, indem spezifische Antikörper gegen eines oder mehrere der Defensinpeptide oder seiner Derivate nach mindestens einem der Ansprüche 1 bis 2 hergestellt werden und die Blutkonzentration eines oder mehrerer der Defensinpeptide nach einem der Ansprüche 1 bis 2 über immunologische Verfahren gemessen wird.

15. Verwendung eines oder mehrerer der Defensinpeptide oder seiner Derivate nach mindestens einem der Ansprüche 1 bis 2 zur Herstellung eines Arzneimittels in verschiedenen galenischen Applikationsformen, insbesondere der lyophilisierten, mit Mannit aufgenommenen Form in sterilen Ampullen zur Auflösung in physiologischer Kochsalzlösung und/oder Infusionslösungen zur wiederholten Einzelinjektion und/oder Dauerinfusion in Mengen von 300 Mikrogramm bis 300 Milligramm eines oder mehrerer der Defensinpeptide nach Anspruch 1 pro Therapie-Einheit.

16. Verwendung der von den Defensinpeptiden nach mindestens einem der Ansprüche 1 bis 2 abgeleiteten Gensonden und Genen zur Herstellung eines Arzneimittels zur lokalen und systemischen Gentherapie der Indikationen gemäß einem der Ansprüche 7 bis 13 in epithelialen Geweben und Organen.

17. Nukleinsäuresequenz codierend für eines oder mehrere der Defensinpeptide oder seine Derivate nach mindestens einem der Ansprüche 1 bis 2.

18. Nukleinsäure mit der Sequenz kodierend für das Defensinpeptid hBD-6.

## Claims

1. A peptide having the amino acid sequence:
(bb) hBD-6
Z_{N3}-CGYGTARCRKKCRSQEYRIGRCPNTYACC-Z_{C3},
wherein Z_{N3} represents an amino acid residue or peptide residue of up to 30 amino acids, especially the peptide residue EFELDRI and its N-terminally truncated fragments, and Z_{C3} represents an amino acid residue or peptide residue of up to 30 amino acids, especially the peptide residue LRKWDESLLNRTKP and its C-terminally truncated fragments.

2. The peptide according to claim 1, wherein said peptide is a cyclic, amidated, acetylated, sulfated, phosphorylated, glycosylated or oxidized derivative derived from the amino acid sequences described in claim 1 and having antibiotic activity.

3. A method for preparing the defensin peptides or their derivatives according to at least one of claims 1 to 2, **characterized in that** they are prepared by prokaryotic or eukaryotic expression and purified.

4. A method for preparing the defensin peptides or their derivatives according to at least one of claims 1 to 2, **characterized in that** the peptides are isolated from human blood in a known way by per se known usual chromatographic methods.

5. A method for preparing the defensin peptides or their derivatives according to at least one of claims 1 to 2, **characterized in that** the defensin peptides or their derivatives are prepared by the usual methods of chemical solid-phase and liquid-phase peptide synthesis from the protected amino acids which are contained in the stated sequences according to claim 1, deblocked and purified by per se known methods.

6. A medicament containing one or more of the defensin peptides or their derivatives according to at least one of claims 1 to 2 as an active ingredient in addition to usual auxiliary agents and additives.

7. Use of one or more of the defensin peptides or their derivatives according to at least one of claims 1 to 2 for preparing a medicament for the treatment of diseases arising from the bacterial colonization of organs.

8. Use of one or more of the defensin peptides or their derivatives according to at least one of claims 1 to 2 for preparing a medicament for the treatment of diseases of the human organism, especially those involving the gastrointestinal tract, the respiratory paths and the urogenital apparatus.

9. Use of one or more of the defensin peptides or their derivatives according to at least one of claims 1 to 2 for preparing a medicament for the treatment of diseases of the human organism, especially those involving the integument and its appendage glands.

10. Use of one or more of the defensin peptides or their derivatives according to at least one of claims 1 to 2 for preparing a medicament for the treatment of systemic diseases when there is an overproduction of or deficiency in the defensin peptides, especially by antibodies formed against the defensin peptides, or for use of the defensin peptides according to at least one of claims 1 to 2 for preparing a medicament for substitution therapy.

11. Use of one or more of the defensin peptides or their derivatives according to at least one of claims 1 to 2 for preparing a medicament for the treatment of chronic diseases which are in part associated with diseases according to claims 7 to 10 by using it in an appropriate form for preparing a medicament for the treatment.

12. Use of one or more of the defensin peptides or their derivatives according to at least one of claims 1 to 2 for preparing a medicament for the treatment of acute diseases according to claims 7 to 11 by using it in an appropriate form for preparing a medicament for the treatment in the intensive care of such diseases.

13. Use of one or more of the defensin peptides or their derivatives according to at least one of claims 1 to 2 for preparing a medicament for the treatment of fertility disorders, especially in diseases involving oocyte-related spermatic penetration disorders and implantation disorders as well as maturation disorders in the male reproduction apparatus, and as a contraceptive.

14. Use of one or more of the defensin peptides or their derivatives according to at least one of claims 1 to 2 for the diagnosis of diseases in vitro, especially those according to claims 7 to 13, by preparing specific antibodies against one or more of the defensin peptides according to at least one of claims 1 to 2 or their derivatives and measuring the blood concentration of one or more of the defensin peptides according to either of claims 1 to 2 by immunological methods.

15. Use of one or more of the defensin peptides or their derivatives according to at least one of claims 1 to 2 for preparing a medicament in different galenic dosage forms, especially in a lyophilized form taken up with mannitol in sterile ampoules for dissolution in physiological saline and/or infusion solutions for repeated singular injection and/or permanent infusion in amounts of from 300 micrograms to 300 milligrams of one or more of the defensin peptides according to claim 1 per therapy unit.

16. Use of the gene probes and genes derived from the defensin peptides according to at least one of claims 1 to 2 for preparing a medicament for the topical and systemic gene therapy of the indications according to any of claims 7 to 13 in epithelial tissues and organs.

17. A nucleic acid sequence coding for one or more of the defensin peptides or their derivatives according to at least one of claims 1 to 2.

18. A nucleic acid sequence having the sequence coding for the defensin peptide hBD-6.

## Revendications

1. Peptide ayant la séquence d'acides aminés suivante :
(bb) hBD-6
Z_{N3}-CGYGTARCRKKCRSQEYRIGRCPNTYACC-Z_{C3}
dans laquelle Z_{N3} représente un résidu d'acide aminé ou un résidu peptidique ayant jusqu'à 30 acides aminés, notamment le résidu peptidique EFELDRI et ses fragments tronqués à l'extrémité N, et Z_{C3} représente un résidu d'acide aminé ou un résidu peptidique ayant jusqu'à 30 acides aminés, notamment le résidu peptidique LRKWDESLLNRTKP et ses fragments tronqués à l'extrémité C.

2. Peptide selon la revendication 1, dans lequel le peptide est un dérivé cyclique, amidé, acétylé, sulfaté, phosphorylé, glycosylé ou oxydé, qui est dérivé des séquences d'acides aminés selon la revendication 1 et qui présente une activité antibiotique.

3. Procédé pour préparer les peptides du genre défensine ou leurs dérivés selon au moins l'une des revendications 1 à 2, **caractérisé en ce que** ceux-ci sont préparés par expression procaryotique ou eucaryotique et purifiés.

4. Procédé pour préparer les peptides du genre défensine ou leurs dérivés selon au moins l'une des revendications 1 à 2, **caractérisé en ce que** les peptides sont isolés de sang humain d'une manière connue par des techniques chromatographiques usuelles connues en soi.

5. Procédé pour préparer les peptides du genre défensine ou leurs dérivés selon au moins l'une des revendications 1 à 2, **caractérisé en ce que** les peptides du genre défensine ou leurs dérivés sont préparés par les techniques usuelles de synthèse chimique de peptides sur phase solide et en phase liquide à partir des acides aminés protégés qui sont compris dans les séquences selon la revendication 1, débloqués et purifiés par des techniques connues en soi.

6. Médicament comprenant un ou plusieurs peptides du genre défensine ou de leurs dérivés selon au moins l'une des revendications 1 à 2 comme ingrédient actif en plus des agents auxiliaires et additifs usuels.

7. Utilisation d'un ou plusieurs des peptides du genre défensine ou de leurs dérivés selon au moins l'une des revendications 1 à 2, pour la fabrication d'un médicament pour le traitement de maladies résultant de la colonisation bactérienne d'organes.

8. Utilisation d'un ou plusieurs des peptides du genre défensine ou de leurs dérivés selon au moins l'une des revendications 1 à 2, pour la fabrication d'un médicament pour le traitement de maladies de l'organisme humain, notamment de celles affectant le tube digestif, les voies respiratoires et l'appareil urogénital.

9. Utilisation d'un ou plusieurs des peptides du genre défensine ou de leurs dérivés selon au moins l'une des revendications 1 à 2, pour la fabrication d'un médicament pour le traitement de maladies de l'organisme humain, notamment de celles affectant le tégument et ses glandes annexes.

10. Utilisation d'un ou plusieurs des peptides du genre défensine ou de leurs dérivés selon au moins l'une des revendications 1 à 2, pour la fabrication d'un médicament pour le traitement de maladies systémiques lorsqu'il existe une surproduction de, ou une carence en, peptides du genre défensine, notamment par des anticorps contre les peptides du genre défensine, ou pour l'utilisation des peptides du genre défensine selon au moins l'une des revendications 1 à 2 pour une thérapie substitutive.

11. Utilisation d'un ou plusieurs des peptides du genre défensine ou de leurs dérivés selon au moins l'une des revendications 1 à 2, pour la fabrication d'un médicament pour le traitement de maladies chroniques qui sont en partie associées à des maladies selon les revendications 7 à 10, en les utilisant sous une forme appropriée pour la fabrication d'un médicament pour le traitement.

12. Utilisation d'un ou plusieurs des peptides du genre défensine ou de leurs dérivés selon au moins l'une des revendications 1 à 2, pour la fabrication d'un médicament pour le traitement de maladies aiguës selon les revendications 7 à 11, en les utilisant sous une forme appropriée pour la fabrication d'un médicament pour le traitement dans les soins intensifs donnés pour ces maladies.

13. Utilisation d'un ou plusieurs des peptides du genre défensine ou de leurs dérivés ou fragments selon au moins l'une des revendications 1 à 2, pour la fabrication d'un médicament pour le traitement de troubles de la fécondité, notamment dans des maladies impliquant des troubles de la pénétration spermatique associés aux oocytes et des troubles d'implantation ainsi que des troubles de maturation dans l'appareil reproducteur mâle, et comme contraceptif.

14. Utilisation d'un ou plusieurs des peptides du genre défensine ou de leurs dérivés selon au moins l'une des revendications 1 à 2, pour le diagnostic in vitro de maladies, notamment de celles selon les revendications 7 à 13, en préparant des anticorps spécifiques dirigés contre un ou plusieurs des peptides du genre défensine ou leurs dérivés selon au moins l'une des revendications 1 à 2, et en mesurant la concentration sanguine d'un ou plusieurs des peptides du genre défensine selon l'une quelconque des revendications 1 à 2 par des techniques immunologiques.

15. Utilisation d'un ou plusieurs des peptides du genre défensine ou de leurs dérivés selon au moins l'une des revendications 1 à 2, pour la fabrication d'un médicament dans différentes formes d'administration galéniques, notamment sous une forme lyophilisée reprise avec du mannitol dans des ampoules stériles destinée à une dissolution dans des solutions salines physiologiques et/ou de perfusion pour une injection séparée répétée et/ou perfusion permanente en des quantités de 300 microgrammes à 300 milligrammes d'un ou plusieurs des peptides du genre défensine selon la revendication 1 par unité thérapeutique.

16. Utilisation de sondes génétiques et gènes dérivés des peptides du genre défensine selon au moins l'une des revendications 1 à 2, pour la fabrication d'un médicament pour la thérapie génique topique et systémique des indications selon l'une quelconque des revendications 7 à 13 dans des tissus épithéliaux et des organes.

17. Séquence d'acide nucléique codant pour un ou plusieurs des peptides du genre défensine ou de leurs dérivés selon au moins l'une des revendications 1 à 2.

18. Une séquence d'acide nucléique ayant la séquence
CGAATTTGAATTGGACAGAATATGTGGTTATGGGACTGCCCGTTGCCGGAA GAA
ATGTCGCAGCCAAGAATACAGAATTGGAAGATGTCCCAACACCTATGCATG CTG
TTTGAGAAAATGGGATGAGAGCTTACTGAATCGTACAAAACCC
codant pour le peptide du genre défensine hBD-6.
